Europäisches Patentamt

European Patent Office

Office européen des brevets

(19)

(11) EP 0 488 889 B1

(12)

# FASCICULE DE BREVET EUROPEEN

(45) Date de publication et mention
de la délivrance du brevet:
**08.07.1998 Bulletin 1998/28**

(51) Int Cl.⁶: **G06T 11/00**

(21) Numéro de dépôt: **91403214.9**

(22) Date de dépôt: **27.11.1991**

(54) **Procédé et dispositif de reconstruction d'images tridimensionnelles d'un objet en utilisant deux trajectoires circulaires d'acquisition**

Verfahren und Vorrichtung zur Bilderzeugung eines Objektes mit zwei Erfassungskreisbahnen

Method and apparatus for reconstructing three-dimensional images of an object using two acquisition circular paths

(84) Etats contractants désignés:
**DE ES GB IT NL SE**

(30) Priorité: **29.11.1990 FR 9014958**

(43) Date de publication de la demande:
**03.06.1992 Bulletin 1992/23**

(73) Titulaire: **COMMISSARIAT A L'ENERGIE ATOMIQUE**
**75015 Paris (FR)**

(72) Inventeurs:
  • **Grangeat, Pierre**
    **F-38330 Saint Ismier (FR)**
  • **Rizo, Philippe**
    **F-38000 Grenoble (FR)**
  • **Sire, Pascal**
    **F-38360 Sassenage (FR)**

(74) Mandataire: **Ilgart, Jean-Christophe et al**
  **c/o Société Brevatome,**
  **25, rue de Ponthieu**
  **F-75008 Paris (FR)**

(56) Documents cités:
  **EP-A- 0 292 402**          **EP-A- 0 379 399**
  **GB-A- 2 088 670**

  • **IEEE.TRANSACTION ON NUCLEAR SCIENCE vol. NS-25, no. 5, Octobre 1978, M.SCLINDWEIN: Iterativethree-dimensional Reconstruction From Twin-Cone Beam Projections.'**
  • **IEEE.TRANSACTION ON NUCLEAR SCIENCE. vol. NS-26, no. 2, Avril 1979, KOWALSKI: 'Multislice Reconstruction from Twin-Cone Scanning'**

## Description

L'invention concerne un procédé et un dispositif de reconstruction d'images tridimensionnelles d'un objet à l'aide d'un réseau bidimensionnel de capteurs parcourant deux trajectoires circulaires d'acquisition des mesures ou, de manière équivalente, deux réseaux bidimensionnels de capteurs parcourant chacun une trajectoire circulaire. Elle peut encore être élargie à un nombre quelconque de trajectoires.

L'image de l'objet est définie par des valeurs prises par une fonction sur chacun de ses points. La fonction est d'autre part une propriété d'un rayonnement (rayons X ou gamma entre autres) de forme conique ayant un point focal et qui passe à travers l'objet ; chaque rayon est reçu par un des capteurs du réseau bidimensionnel et représente donc la somme de la fonction sur tous les points de l'objet appartenant à ce rayon. Un traitement approprié des sommes sur tous les rayons, et ceci pour un nombre suffisant de mesures suivant des incidences différentes autour de l'objet, permet de reconstituer l'image de l'objet.

On se contente en pratique de considérer des nombres finis de rayons et de points de l'objet selon des discrétisations ou des maillages.

Cette invention constitue un perfectionnement d'une invention précédente, décrite dans la demande de brevet européen EP-A-0 292 402, mais on peut envisager d'employer l'invention actuelle dans d'autres circonstantes ou en utilisant d'autres méthodes mathématiques de traitement des données.

Les méthodes envisageables utilisent en particulier ce qu'on appelle la transformée de Radon de la fonction à mesurer, qui est définie comme la somme de la fonction sur chacun des plans, appelés plans de Radon, qui passent à travers l'objet considéré ou, mieux encore, la dérivée première de cette transformée. La contribution des points de ces plans qui n'appartiennent pas à l'objet est considérée comme inexistante, ce qui est valable dans le cas d'un rayonnement qui traverse un milieu gazeux ambiant sans être atténué. Ici encore, une discrétisation est effectuée pour n'effectuer les calculs que sur un nombre fini de plans.

La dérivée première de la transformée de Radon est définie comme la dérivée de la transformée de Radon en fonction de la variable $\rho$ définissant la distance du plan considéré à une origine. Pour chaque plan, elle correspond à la somme sur ce plan de la dérivée première de la transformée de Radon dans la direction perpendiculaire au plan. L'invention décrite dans le brevet EP-A-0 290 402 montre que pour un plan passant par une position du point focal du cône de rayonnement et rencontrant le réseau bidimensionnel de capteurs, il est possible de calculer à partir des mesures la valeur exacte de la dérivée première de la transformée de Radon sur ce plan. Ce calcul, suivant les formules exposées dans le brevet EP-A-0 292 402, met en oeuvre de façon préférentielle une pondération de correction d'éloignement du point focal, deux filtrages correspondant aux calculs des dérivés premières respectivement suivant les lignes et les colonnes du réseau de capteurs, deux sommations suivant la droite d'intersection entre le plan à traiter et le réseau bidimensionnel de capteurs, puis une combinaison linéaire et une normalisation des résultats. Cette sommation met en oeuvre les interpolations nécessaires car ces droites d'intersection passent entre les rangées de capteurs ou les coupent.

Dans le reste de la demande, on entend par somme la somme pondérée des valeurs mesurées (dans le cas de la transformée de Radon) aussi bien que la combinaison linéaire des sommes des valeurs pondérées et filtrées (dans le cas de la dérivée première de la transformée de Radon) obtenues selon les lignes et les colonnes du réseau.

La somme de la fonction sur les points des plans de Radon est facile à obtenir à condition que ces plans aient une intersection avec le réseau bidimensionnel de capteurs et qu'ils passent par le point focal unique que visent les capteurs. Il suffit d'effectuer la somme des valeurs mesurées par chaque capteur situé à l'intersection, avec les interpolations nécessaires car les intersections des plans de Radon passent entre des rangées de capteurs ou les coupent. Une fois que les valeurs de la fonction sur les plans de Radon ont été calculées, il existe des formules d'inversion, exposées dans la demande de brevet mentionnée ci-dessus, qui permettent de parvenir aux valeurs de la fonction sur les points du maillage de l'objet correspondant à l'image à reconstruire.

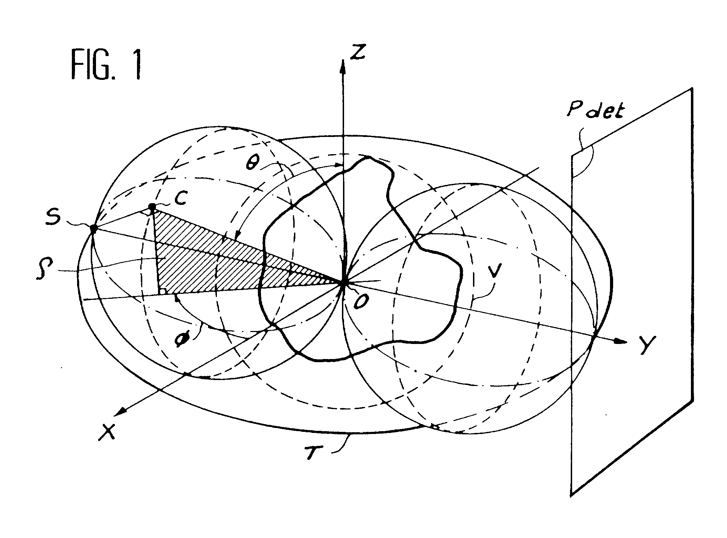

Il faut toutefois revenir sur les conditions qui permettent d'obtenir un nombre de plans de Radon suffisant pour permettre une description satisfaisante de l'objet. Chaque plan de Radon peut être défini par ce qu'on appelle son point caractéristique, c'est-à-dire le point de projection sur ce plan d'une origine O choisie arbitrairement. Ce point caractéristique, noté C sur la figure 1, peut être défini par ses coordonnées sphériques $\rho$, $\phi$ et $\theta$ de rayon, longitude et colatitude respectivement à partir de l'origine O. Le plan de Radon P passant par le point caractéristique C peut être défini par le rayon $\rho$ et le vecteur unitaire $\vec{n}$ de direction $\overrightarrow{OC}$.

Les valeurs de la fonction sur les plans de Radon ne peuvent effectivement être calculées que pour les plans de Radon qui coupent la trajectoire parcourue par le point focal du rayonnement. Ce point focal est concrètement, dans le cas d'une fonction d'atténuation, une source ponctuelle de rayons X, gamma, etc. C'est la conception qui est décrite dans la demande de brevet européenne mentionnée. Les mêmes conditions géométriques existent en tomographie d'émission, quand la fonction à mesurer est l'activité émise par le corps. Le point focal n'a alors pas d'existence physique et correspond simplement au point de convergence vers lequel tous les collimateurs utilisés devant le réseau bidimensionnel sont dirigés.

Il faut, pour obtenir un nombre suffisant de plans de Radon, effectuer plusieurs mesures avec des positions différentes du point focal.

Considérons une trajectoire circulaire T parcourue par le point focal (ou la source) S, le réseau plan de capteurs Pdét parcourant la même trajectoire que le point focal S ou éventuellement une trajectoire concentrique de rayon différent. Supposons encore que l'origine O qui sert à définir les points caractéristiques C soit confondue avec le centre de la trajectoire T. Le volume enveloppant les points caractéristiques correspond à un tore To produit par la rotation d'une surface sphérique de diamètre OS autour de l'axe de rotation de la trajectoire T. En effet, les plans de Radon qui passent par le point focal S ont leurs points caractéristiques répartis sur la surface sphérique de diamètre OS car l'angle $\widehat{SCO}$ est droit. Le tore To est appelé le volume caractéristique des mesures, qui dépend donc de la forme de la trajectoire T et de sa position par rapport à l'origine O.

Il est par ailleurs suffisant, pour obtenir une description complète de l'objet par les procédés utilisant les sommes de la fonction sur les plans de Radon, de pouvoir disposer des points caractéristiques appartenant à un volume caractéristique de l'objet. Ce volume caractéristique de l'objet est toujours inclus dans une sphère V centrée sur l'origine O et qui englobe l'objet M. On peut donc assurer que si le volume caractéristique de l'objet est inclus dans le volume caractéristique des mesures, la reconstruction de l'image de l'objet M sera possible. Dans le cas d'une trajectoire circulaire T, cette condition n'est malheureusement pas remplie car une zone d'ombre subsiste pour les points caractéristiques de la sphère V qui n'appartiennent pas au tore To et dont les plans de Radon ne coupent pas la trajectoire T.

La demande de brevet précédente contient la constatation que le choix de certaines trajectoires plus compliquées permet d'agrandir le volume caractéristique des mesures pour remplir complètement le volume caractéristique de l'objet. Un cas intéressant est celui de la superposition de deux trajectoires circulaires concentriques et appartenant à des plans différents, par exemple perpendiculaires, car la superposition des deux tores concentriques permet d'éliminer la zone d'ombre autour de l'origine. Un dispositif adapté à de telles mesures comprend un rail ou une glissière matérialisant la trajectoire T, sur lequel un émetteur de rayons X et le réseau bidimensionnel de capteurs circulent de manière à être toujours diamétralement opposés, et qui peut basculer autour d'un axe fixe. Aucune indication n'avait toutefois été donnée sur la façon de reconstruire effectivement l'image de l'objet M à partir des deux séries de mesures et de leur synthèse. La présente invention a précisément pour sujet un procédé adapté à la reconstruction d'images au moyen de mesures prises suivant deux trajectoires circulaires, sans qu'aucune condition ne soit exigée sur les relations géométriques entre ces trajectoires.

Pour récapituler, l'invention concerne un procédé de reconstruction d'images tridimensionnelles conforme à la revendication 1 et un dispositif conforme à la revendication 12.

Ainsi, chaque série de mesures de la fonction peut être calculée d'abord avec les paramètres (incidence de la mesure, distance entre le point focal et le réseau, orientation du réseau par rapport à l'axe de la trajectoire...) spécifiques à la série de mesures correspondante avant de se placer dans des conditions homogènes au moment du regroupement des mesures.

Avantageusement, les repères de trajectoire et d'objet ont un axe de même direction, ce qui est toujours possible dans le cas où il n'y a que deux trajectoires, ou, encore, les repères de trajectoire ont un axe confondu avec l'axe de la trajectoire circulaire correspondante ; de préférence, l'origine des repères de trajectoire est confondue avec le centre du cercle de la trajectoire.

Il est encore avantageux que les groupes de coordonnées dans les repères de trajectoire et le repère d'objet soient des coordonnées sphériques et que le calcul de changement de repère comporte une conversion temporaire des coordonnées sphériques en coordonnées cartésiennes.

Il est encore possible que le repère d'objet coïncide avec un des repères de trajectoire.

Un cas particulier important est celui de trajectoires concentriques.

Le repère d'objet aura de préférence un axe suivant une direction du maillage décrivant l'image de l'objet.

Le dispositif selon l'invention comprend au moins un réseau bidimensionnel de capteurs, les réseaux étant déplacés autour d'un objet à examiner suivant au moins deux trajectoires circulaires et reliés à des moyens d'acquisition de mesures et de traitement des mesures appropriés à la mise en oeuvre du procédé de l'invention.

On va maintenant décrire l'invention à l'aide des figures suivantes annexées à titre illustratif et non limitatif :

- la figure 1, déjà décrite, résume les conditions géométriques à la source de l'invention ;
- la figure 2 représente un dispositif permettant la mise en oeuvre pratique de l'invention ;
- la figure 3 illustre les moyens géométriques mis en oeuvre ;
- la figure 4 est une explication d'une étape d'interpolation ; et
- les figures 5 et 6 représentent un autre dispositif possible.

Dans le cas où l'objet à examiner est un corps humain irradié par une source externe, le patient peut être allongé sur une table 1. Un rail circulaire 2 entoure la table 1 et peut être mis en rotation autour d'un axe vertical à l'aide de deux charnières 3 qui le relient à des points fixes de l'installation. Le rail circulaire 2 est en fait une crémaillère qui porte en des positions diamétralement opposées une source 4 émettrice d'un faisceau conique 6 de rayons X et un écran 5 porteur d'un réseau bidimensionnel de capteurs. La source 4 et l'écran 5 sont déplacés le long du rail 2 de conserve sous l'action de moteurs non représentés commandés automatiquement et qui actionnent des pignons engrenant avec la crémaillère. Les trajectoires circulaires différentes sont obtenues par la rotation du rail 2. Il est encore possible d'avoir un rail fixe et une table 1 mobile entre des positions déterminées à l'avance. Il existe bien d'autres dispositifs qui peuvent être utilisés, et dont quelques-uns sont décrits dans la demande de brevet antérieure.

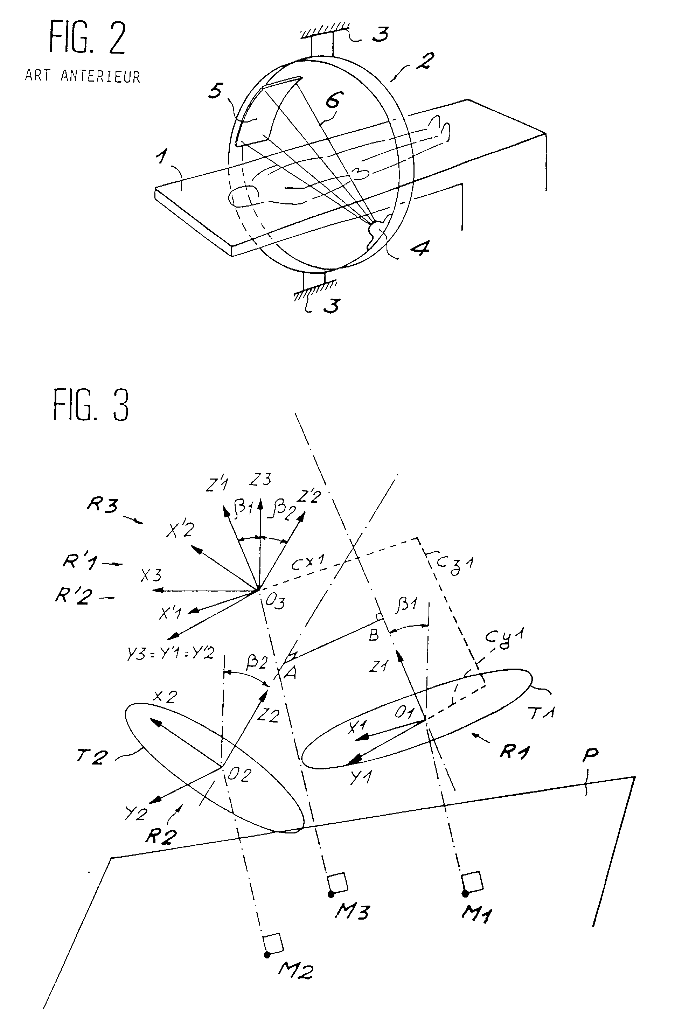

La figure 3 représente le mode de mise en oeuvre du procédé dans le cas le plus général où deux trajectoires circulaires T1 et T2 ont des axes non concourants et non parallèles et dont les directions sont quelconques. AB désigne le segment le plus court pour joindre ces axes. Les repères de trajectoire R1 et R2 ont pour origine les centres O1 et O2 des trajectoires T1 et T2, les axes Z1 et Z2 sont perpendiculaires aux trajectoires respectives et les axes Y1 et Y2 sont parallèles. On définit en outre un repère d'objet R3 dans lequel les reconstructions de l'image de l'objet sont effectuées. L'axe Y3 de ce repère est parallèle aux axes Y1 et Y2 pour faciliter les calculs. Aucune autre condition n'est requise pour que les calculs qui vont suivre soient applicables. En pratique, les origines O1, O2 et O3 seront généralement confondues et le repère R3 sera identique à un des autres repères R1 ou R2, mais comme ces cas simplificateurs ne sont pas considérés ici par souci de généralité, il faut donc en outre définir deux repères intermédiaires R'1 et R'2 d'origine O3 et dont les axes sont parallèles à ceux des repères de trajectoire R1 et R2 respectivement. On désigne par β1 et β2 les angles entre l'axe Z3 d'une part, les axes Z1 ou Z'1 et Z2 ou Z'2 d'autre part.

Soit maintenant un plan de Radon P sur lequel le calcul de somme a été effectué et pour lequel un changement de repère est nécessaire pour effectuer le regroupement ou la fusion des séries de mesures. Les projections orthogonales des trois origines O1, O2 et O3 sur le plan P sont désignées par M1, M2 et M3. Le point M3 est le point caractéristique du plan de Radon dans le repère d'objet R3 et donc essentiel pour les calculs d'inversion de la transformée de Radon qui mènent à la reconstruction de l'image de l'objet. Il n'a cependant aucune signification dans les repères de trajectoire R1 et R2 où le même plan de Radon P est défini par les points caractéristiques M1 ou M2.

Les points M1 à M3 sont définis généralement par leurs coordonnées sphériques car ces coordonnées sont les plus pratiques pour définir des maillages réguliers sur des volumes mesurés à l'aide de trajectoires circulaires ainsi que pour définir les points caractéristiques. Le passage en coordonnées cartésiennes n'est en principe effectué qu'à l'étape finale, pour définir des images de l'objet par des plans de coupe. Une conversion temporaire en coordonnées cartésiennes est cependant effectuée dans le procédé décrit ici. Si i est l'indice d'un des repères R1, R2, ou R3, on dispose des formules suivantes :

$$X_i = \rho_i \sin(\theta_i) \cos(\phi_i)$$

$$Y_i = \rho_i \sin(\theta_i) \sin(\phi_i)$$

$$Z_i = \rho_i \cos(\theta_i)$$

pour assurer la correspondance avec des coordonnées cartésiennes qui seront utilisées dans les calculs de changement de repère.

Les formules suivantes :

$$X1 = X'1 \cdot \left(1 + \frac{(cx1.X'1 + cy1.Y'1 + cz1.Z'1)}{X'1^2 + Y'1^2 + Z'1^2}\right)$$

$$Y1 = Y'1 \cdot \left(1 + \frac{cx1.X'1 + cy1.Y'1 + cz1.Z'1}{X'1^2 + Y'1^2 + Z'1^2}\right)$$

$$Z1 = Z'1 \cdot \left(1 + \frac{cx1.X'1 + cy1.Y'1 + cz1.Z'1}{X'1^2 + Y'1^2 + Z'1^2}\right)$$

$$X'_1 = X_3 \cos(\beta_1) + Z3 \sin(\beta_1)Y$$

$$Y'_1 = Y_3$$

$$Z'_1 = -X_3 \sin(\beta_1) + Z3 \cos(\beta_1)$$

où cx1, cy1 et cz1 sont les coordonnées cartésiennes de l'origine O3 dans le repère R1 de trajectoire, permettent de retrouver les coordonnées X1, Y1 et Z1 du point M1 dans le repère de trajectoire R1 en fonction des coordonnées X3, Y3 et Z3 du point M3 dans le repère d'objet R3. Des transformations inverses seraient également possibles. Le calcul pour le point M2 est identique à un changement d'indice près.

On calcule ensuite les coordonnées sphériques $\theta1$, $\rho1$ et $\phi1$ du point M1 dans le repère R1 par les formules suivantes, où "atan" signifie arc tangente :

si $Z_1 > 0$ :

$$\theta_1 = \operatorname{atan}(\sqrt{X_1^2 + Y_1^2} \, / \, Z_1)$$

$$\rho_1 = \sqrt{X_1^2 + Y_1^2 + Z_1^2}$$

si $Z_1 < 0$ :

$$\theta_1 = \operatorname{atan}(\sqrt{X_1^2 + Y_1^2} \, / \, (-Z_1))$$

$$\rho_1 = - \sqrt{X_1^2 + Y_1^2 + Z_1^2}$$

si $Z_1 = 0$ :

$$\theta_1 = \pi/2$$

$$\rho_1 = \sqrt{X_1^2 + Y_1^2 + Z_1^2}$$

si $Z_1 > 0$ :
    si $Y_1 > 0$ :

$$\phi_1 = \operatorname{acos}(X_1 \, / \, \sqrt{X_1^2 + Y_1^2})$$

    si $Y_1 < 0$ :

$$\phi_1 = 2\pi - \operatorname{acos}(X_1 \, / \, \sqrt{X_1^2 + Y_1^2})$$

    si $Y_1 = 0$ :
    si $X_1 > 0$ :

$$\phi_1 = 0$$

    si $X_1 < 0$ :

$$\phi_1 = \pi$$

si $X_1 = 0$ :

$$\phi_1 = 0.$$

si $Z_1 \leqq 0$ :
si $Y_1 > 0$ :

$$\phi_1 = \pi + \text{acos}(X_1 / \sqrt{X_1{}^2 + Y_1{}^2})$$

si $Y_1 < 0$ :

$$\phi_1 = \pi - \text{acos}(X_1 / \sqrt{X_1{}^2 + Y_1{}^2})$$

si $Y_1 = 0$ :
si $X_1 > 0$ :

$$\phi_1 = \pi$$

si $X_1 < 0$ :

$$\phi_1 = \pi$$

si $X_1 = 0$ :

$$\phi_1 = 0.$$

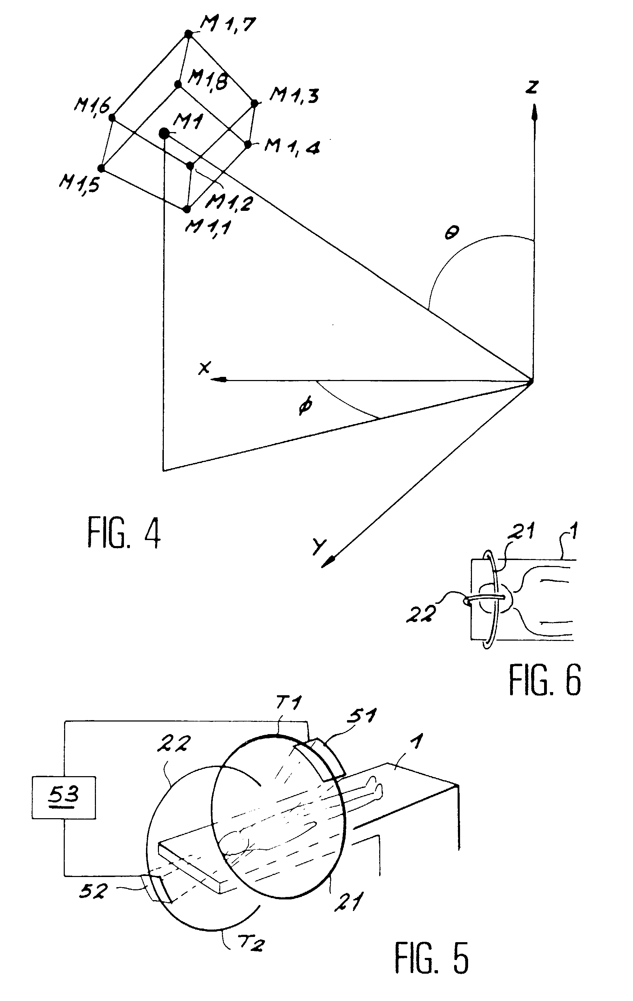

Cependant, le point M1 ainsi obtenu n'est normalement pas un point d'échantillonnage du volume de Radon correspondant à la première trajectoire T1, mais un point intermédiaire entre les points du maillage retenu. On effectue alors une interpolation du maillage dans un volume pour affecter une valeur au point M1 (ou $M_i$ dans le cas général) à partir de huit points environnants $M_{i1}$ à $M_{i8}$, dont les valeurs de la somme de la fonction sur le plan de Radon associé sont $V_{i,j}$. Les coefficients d'interpolation sont $d\rho i$, $d\theta i$ et $d\phi i$ et les pas d'échantillonnage sont $pe\rho$, $pe\theta$ et $pe\phi$ selon les conventions de la figure 4. On applique les formules suivantes :

$$d\rho_i = (\rho_i - \rho_{i,1})/pe\rho = (\rho_i - \rho_{i,2})/pe\rho = (\rho_i - \rho_{i,3})/pe\rho = (\rho_i - \rho_{i,4})/pe\rho$$

$$d\theta_i = (\theta_i - \theta_{i,1})/pe\theta = (\theta_i - \theta_{i,2})/pe\theta = (\theta_i - \theta_{i,7})/pe\theta = (\theta_i - \theta_{i,6})/pe\theta$$

$$d\phi_i = (\phi_i - \phi_{i,1})/pe\phi = (\phi_i - \phi_{i,4})/pe\phi = (\phi_i - \phi_{i,6})/pe\phi = (\phi_i - \phi_{i,6})/pe\phi$$

où $\rho_{ij}$, $\theta_{ij}$ et $\phi_{ij}$ sont les coordonnées sphériques du point $M_{ij}$,
puis :
(interpolation sur $\rho$)

$$I_{i,1} = Vc_{i,1}(1 - d\rho_i) + Vc_{i,5}d\rho_i$$

$$I_{i,2} = Vc_{i,2}(1 - d\rho_i) + Vc_{i,6}d\rho_i$$

$$I_{i,3} = Vc_{i,3}(1-d\rho_i) + Vc_{i,7}d\rho_i$$

$$I_{i,4} = Vc_{i,4}(1-d\rho_i) + Vc_{i,8}d\rho_i$$

(Interpolation sur $\theta$)

$$\Pi_{i,1} = I_{i,1}(1-d\theta_i) + I_{i,4}d\theta_i$$

$$\Pi_{i,2} = I_{i,2}(1-d\theta_i) + I_{i,3}d\theta_i$$

(Interpolation sur $\phi$)

$$V_i = \Pi_{i,1}(1-d\phi i) + \Pi_{i,2}d\phi_i.$$

Dans le cas où la fonction considérée est la dérivée première de la transformée de Radon, dont le signe dépend du sens de parcours des rayons $\rho$ correspondant aux points M, on définit $V_{ci,j} = V_{i,j}$ si $\rho i,j$ a le même signe que $\rho 3$ ; sinon, $V_{ci,j} = -V_{i,j}$. On rappelle que les coordonnées sphériques sont définies avec $-\infty < \rho < +\infty$, $0 < \theta < \frac{\pi}{2}$ et $0 < \phi < 2\pi$.

Pour chacun des points du volume caractéristique de l'objet défini dans le repère d'objet, le regroupement consiste, par exemple sur un point de l'intersection des volumes caractéristiques de chacune des trajectoires, à effectuer une moyenne des sommes de fonction associées à chaque trajectoire ou, pour un point appartenant exclusivement à l'un des volumes caractéristiques des trajectoires, à considérer la somme de la fonction calculée dans le repère de cette trajectoire, ces volumes caractéristiques étant définis par rapport à l'origine du repère de l'objet.

Les calculs d'inversion, conformément à la demande de brevet antérieur par exemple, sont effectués quand des sommes de valeurs ont été réunies pour tous les points correspondant au volume caractéristique de l'objet. Les mêmes étapes de filtrage, de pondération et de rétroprojection sont effectuées.

Le procédé peut aussi être employé avec des modélisations différentes ou des repères différents, et on peut utiliser plus de deux trajectoires circulaires.

Par ailleurs, on a considéré avant tout l'utilisation de ce procédé pour l'inversion de la transformée de Radon proprement dite ou de sa dérivée première. On peut encore procéder sensiblement de la même façon avec une transformée de Fourier de tous les rayons de l'espace de Radon quine coupent pas la zone d'ombre, et ceci pour chacun des volumes caractéristiques de mesure. La synthèse des mesures ainsi transformées dans le troisième repère peut être menée sans changement pour peu que les repères R1, R2 et R3 aient leurs centres confondus.

Au lieu de la transformée de Radon ou de ses dérivées premières, on peut encore procéder de la même façon avec la transformée de Hilbert de ces transformées.

Le filtrage de la dérivée première pourrait également être effectué avant de réaliser l'interpolation de la figure 4, ce qui permettrait de ne pas déterminer les orientations. Par contre, il faudrait effectuer le filtrage des dérivées une fois pour chaque volume R1 ou R2.

Si les mesures prises autour des trajectoires T1 et T2 sont en nombre insuffisant, plutôt que de recourir à une interpolation de réarrangement, exposée dans la demande de brevet précédente, où les plans de Radon représentatifs des points M1 ou M2 ne sont pas mesurés directement mais à partir d'interpolations réalisées sur des mesures correspondant à deux positions voisines du point focal S, il est préférable d'incorporer l'opération de réarrangement dans la phase de regroupement décrite dans la présente demande, afin de n'avoir à réaliser qu'une seule interpolation.

Comme il a déjà été signalé, l'invention peut aussi être utilisée en tomographie d'émission. On peut alors plus facilement accepter des trajectoires circulaires incomplètes. Comme les figures 5 et 6 le montrent, un autre dispositif comporte un premier rail 21 entourant la table 1 et appartenant à un plan transversal par rapport au patient. Un second rail 22 est situé à une extrémité de la table 1 et appartient à un plan longitudinal du patient ; contrairement au précédent, il est interrompu pour livrer passage à la table 1 et au patient. Une telle disposition est intéressante par exemple pour l'examen du cerveau.

Un écran 51 parcourt une trajectoire T1 en cercle complet autour du rail 21 et un écran 52 parcourt une trajectoire T2 en arc de cercle autour du rail 22. Comme toutefois il n'y a pas de source en tomographie d'émission, le point focal S opposé à l'écran 52 sur la trajectoire T2 peut valablement être situé sur la zone d'interruption du rail 22. Les moyens d'acquisition et de traitement des mesures portent la référence générale 53.

L'invention peut servir pour les applications usuelles d'imagerie médicale ou de contrôle non destructif de pièces.

## EP 0 488 889 B1

**Revendications**

1. Procédé de reconstruction d'images tridimensionnelles d'un objet (M) défini par des valeurs prises par une fonction sur des points de l'objet, la fonction étant une propriété de l'objet exprimée par un rayonnement conique émis par l'objet ou atténué par l'objet, ayant un point focal (S) et passant à travers l'objet (M), dans lequel la fonction est calculée par un algorithme d'inversion utilisant des sommes de la fonction calculées sur des groupes desdits points appartenant à des plans (P) passant par au moins un point de l'objet et le point focal (S) et définis par des coordonnées exprimées dans un repère d'objet (R3), comportant au moins deux séries de mesures, chaque somme de la fonction étant calculée, sur lesdits plans de l'objet, à partir d'au moins une des séries de mesures, chacune des séries de mesures étant menée avec un réseau bidimensionnel de capteurs (5) de rayonnement orientés vers le point focal, qui est déplacé autour de l'objet sur une trajectoire circulaire ($T_1$, $T_2$) respective, procédé caractérisé en ce que, pour chaque série de mesures, une série des sommes de la fonction est calculée sur un sous-groupe respectif desdits plans, les plans dudit sous-groupe étant sécants ou tangents à la trajectoire associée à ladite série de mesures et étant alors définis par des coordonnées exprimées dans un repère de trajectoire respectif associé à ladite trajectoire, et en ce que les séries de sommes de la fonction sont regroupées en une série unique exprimant lesdites séries de sommes dans le repère d'objet avant d'appliquer l'algorithme d'inversion en effectuant des calculs pour pouvoir définir les plans par des coordonnées exprimées dans le repère d'objet.

2. Procédé de reconstruction d'images tridimensionnelles suivant la revendication 1, caractérisé en ce que les repères (R1, R2, R3) de trajectoire et d'objet ont un axe de même direction (Y1, Y2, Y3).

3. Procédé de reconstruction d'images tridimensionnelles suivant l'une quelconque des revendications 1 ou 2, caractérisé en ce que les repères de trajectoire ont un axe (Z1, Z2) perpendiculaire à la trajectoire respective (T1, T2).

4. Procédé de reconstruction d'images tridimensionnelles suivant l'une quelconque des revendications 1 à 3, caractérisé en ce que les coordonnées dans les repères de trajectoire et le repère d'objet sont des coordonnées sphériques, et en ce que les calculs de changement de repère comportent une conversion temporaire des coordonnées sphériques ($\rho$, $\theta$, $\phi$) en coordonnées cartésiennes (X, Y, Z).

5. Procédé de reconstruction d'images tridimensionnelles suivant l'une quelconque des revendications 1 à 4, caractérisé en ce que le repère d'objet coïncide avec un des repères de trajectoire.

6. Procédé de reconstruction d'images tridimensionnelles suivant l'une quelconque des revendications 1 à 5, caractérisé en ce que les trajectoires sont concentriques.

7. Procédé de reconstruction d'images tridimensionnelles suivant l'une quelconque des revendications 1 à 6, caractérisé en ce que les plans sont définis dans les repères de trajectoire et d'objet par des points caractéristiques appartenant à ces plans, les points caractéristiques associés aux repères de trajectoire et d'objet étant différents, et en ce que les opérations de regroupement comportent une conversion entre les coordonnées dans le repère de trajectoire (X1, Y1, Z1) du point caractéristique (M1) associé audit repère de trajectoire (R1) et les coordonnées (X3, Y3, Z3) dans le repère d'objet (R3) du point caractéristique (M3) associé au repère d'objet.

8. Procédé de reconstruction d'images tridimensionnelles suivant la revendication 7, caractérisé en ce que les opérations de regroupement comportent, après la conversion, une moyenne des sommes de fonction associées à différentes trajectoires pour certains des points caractéristiques associés au repère d'objet.

9. Procédé de reconstruction d'images tridimensionnelles suivant l'une quelconque des revendications 1 à 7, caractérisé en ce que le rayonnement est un rayonnement d'atténuation émis par une source (4) ponctuelle au point focal, tournant autour de l'objet suivant une trajectoire circulaire de source.

10. Procédé de reconstruction d'images tridimensionnelle suivant l'une quelconque des revendications 1 à 7, caractérisé en ce que le rayonnement est un rayonnement d'émission provenant de l'objet, le point focal étant un point vers lequel convergent les directions de mesure des capteurs.

11. Procédé de reconstruction d'images tridimensionnelles suivant l'une quelconque des revendications 1 à 10, caractérisé en ce que le repère d'objet a un axe correspondant à une direction d'un maillage de discrétisation de l'image tridimensionnelle de l'objet.

12. Dispositif comprenant au moins un réseau bidimensionnel de capteurs, le réseau étant déplacé autour d'un objet à examiner suivant au moins deux trajectoires circulaires et relié à des moyens d'acquisition de mesures et de traitement des mesures appropriés à la mise en oeuvre d'un procédé conforme à l'une quelconque des revendications précédentes.

**Patentansprüche**

1. Verfahren zur Erzeugung dreidimensionaler Bilder eines Objekts (M), definiert durch Werte, die mittels einer Funktion an Punkten des Objekts abgenommen bzw. gemessen werden, wobei die Funktion eine Eigenschaft des Objekts ist, ausgedrückt durch eine konische Strahlung, abgestrahlt durch das Objekt oder gedämpft durch das Objekt, die einen Fokalpunkt (S) hat und das Objekt (M) durchquert, bei dem die Funktion durch einen Inversionsalgorithmus berechnet wird, indem Summen der Funktion benutzt werden, berechnet aufgrund von Gruppen der genannten Punkte, zu Ebenen (P) gehörend, die wenigstens einen Punkt des Objekts und den Fokalpunkt (S) durchlaufen und definiert werden durch in einem Objektbezugssystem (R3) ausgedrückte Koordinaten, wenigstens zwei Meßreihen umfassend, wobei jede Summe der Funktion auf den genannten Ebenen des Objekts berechnet wird aufgrund wenigstens einer der Meßreihen, jede der Meßreihen durchgeführt wird mit einem zweidimensionalen Strahlungssensorengitter (5), ausgerichtet auf den Fokalpunkt, der in einer jeweils kreisförmigen Bahn ($T_1$, $T_2$) um das Objekt herum bewegt wird,
   **dadurch gekennzeichnet,**
   daß für jede Meßreihe eine Summenreihe der Funktion aufgrund jeweils einer Untergruppe der genannten Ebenen berechnet wird, die Ebenen der genannten Untergruppe die Bahn schneiden oder tangieren, die der genannten Meßreihe zugeordnet ist, und dann durch Koordinaten definiert werden, die in einem der genannten Bahn zugeordneten Bahnbezugssystem ausgedrückt werden, und dadurch, daß vor Anwendung des Inversionsalgorithmus die Summenreihen der Funktion zu einer einzigen Reihe zusammengefaßt werden, die die genannten Summenreihen in dem Objektbezugssystem ausdrückt, vor Anwendung des Inversionsalgorithmus, indem Berechnungen durchgeführt werden, um die Ebenen durch in dem Objektbezugssystem ausgedrückte Koordinaten definieren zu können.

2. Verfahren nach Anspruch 1, dadurch gekennzeichnet, daß die Bahn- und Objektbezugssysteme (R1, R2, R3) eine Achse derselben Richtung (Y1, Y2, Y3) haben.

3. Verfahren nach einem der Ansprüche 1 oder 2, dadurch gekennzeichnet, daß die Bahnbezugssysteme eine zur jeweiligen Bahn (T1, T2) senkrechte Achse (Z1, Z2) haben.

4. Verfahren nach einem der Ansprüche 1 bis 3, dadurch gekennzeichnet, daß die Koordinaten in den Bahnbezugssystemen und den Objektbezugssystemen Kugelkoordinaten sind, und dadurch, daß die Bezugssystemänderungsberechnungen eine temporäre Umwandlung der Kugelkoordinaten ($\rho$, $\theta$, $\phi$) in Punktkoordinaten umfaßt.

5. Verfahren nach einem der Ansprüche 1 bis 4, dadurch gekennzeichnet, daß das Objektbezugssystem mit einem der Bahnbezugssysteme zusammenfällt.

6. Verfahren nach einem der Ansprüche 1 bis 5, dadurch gekennzeichnet, daß die Bahnen konzentrisch sind.

7. Verfahren nach einem der Ansprüche 1 bis 6, dadurch gekennzeichnet, daß die Ebenen in den Bahn- und Objektbezugssystemen durch charakteristische Punkte definiert werden, die zu diesen Ebenen gehören, wobei die den Bahn- und Objektbezugssystemen zugeordneten Punkte verschieden sind, und dadurch, daß die Umgruppierungs- bzw. Zusammenfassungsoperationen in dem Bahnbezugssystem (X1, Y1, Z1) des zu dem genannten Bahnbezugssystem (R1) gehörenden charakteristischen Punkts (M1) und der Koordinaten (X3, Y3, Z3) in dem Objektbezugssystem (R3) des zum Objektbezugssystem gehörenden charakteristischen Punkts (M3) eine Umwandlung unter den Koordinaten umfassen.

8. Verfahren nach Anspruch 7, dadurch gekennzeichnet, daß die Umgruppierungs- bzw. Zusammenfassungsoperationen nach der Umwandlung für bestimmte dem Objektbezugssystem zugeordnete charakteristische Punkte einen Mittelwert der verschiedenen Bahnen zugeordneten Funktionssummen umfassen.

9. Verfahren nach einem der Ansprüche 1 bis 7, dadurch gekennzeichnet, daß die Strahlung eine von einer punktförmigen Fokalpunkt-Quelle (4) stammende Dämpfungsstrahlung ist, die sich in einer kreisförmigen Bahn um das Objekt herum dreht.

10. Verfahren nach einem der Ansprüche 1 bis 7, dadurch gekennzeichnet, daß die Strahlung eine vom Objekt kommende Emissionsstrahlung ist, wobei der Fokalpunkt ein Punkt ist, gegen den die Richtungen der Meßsensoren konvergieren.

11. Verfahren nach einem der Ansprüche 1 bis 10, dadurch gekennzeichnet, daß das Objektbezugssystem eine Achse hat, die einer Richtung eines Aufgliederungsnetzwerks des dreidimensionalen Bilds des Objekts entspricht.

12. Vorrichtung mit wenigstens einem zweidimensionalen Sensorengitter, wobei das Gitter in wenigstens zwei kreisförmigen Bahnen um ein zu untersuchendes Objekt herum bewegt wird und mit Erfassungseinrichtungen und Verarbeitungseinrichtungen von Messungen verbunden ist, die geeignet sind für die Durchführung des Verfahrens nach einem der vorangehenden Ansprüche.

## Claims

1. Process for the reconstruction of three-dimensional images of an object (M) defined by values assumed by a function on points of the object, the function being a property of the object expressed by a conical radiation emitted or attenuated by the object, having a focal point (S) and passing through the object (M), in which the function is calculated by an inversion algorithm using sums of the function calculated on groups of said points belonging to planes (P) passing through at least one point of the object and the focal point (S) and defined by coordinates expressed in an object reference frame (R3), having at least two series of measurements, each sum of the function being calculated, on said planes of the object, from at least one of the series of measurements, each of the series of measurements being performed with a bidimensional array of radiation sensors (5) oriented towards the focal point, which is displaced about the object on a respective circular path ($T_1$, $T_2$), said process being characterized in that, for each series of measurements, a series of sums of the function is calculated on a respective subgroup of said planes, the planes of said subgroup being secant or tangential to the trajectory associated with said series of measurements and then being defined by coordinates expressed in a respective path reference frame associated with said path and in that the series of sums of the function are pooled in a single series expressing said series of sums in the object reference frame before applying the inversion algorithm, whilst performing calculations in order to be able to define the planes by the coordinates expressed in the object reference frame.

2. Process for the reconstruction of three-dimensional images according to claim 1, characterized in that the path and object reference frames (R1, R2, R3) have an axis with the same direction (Y1, Y2, Y3).

3. Process for the reconstruction of three-dimensional images according to claims 1 or 2, characterized in that the path reference frames have an axis (Z1, Z2) perpendicular to the respective path (T1, T2).

4. Process for the reconstruction of three-dimensional images according to any one of the claims 1 to 3, characterized in that the groups of coordinates in the path reference frames and the object reference frame are spherical coordinates and in that the reference frame change calculations involve a temporary conversion of spherical coordinates ($\rho$, $\theta$, $\phi$) into cartesian coordinates (X, Y, Z).

5. Process for the reconstruction of three-dimensional images according to any one of the claims 1 to 4, characterized in that the object reference frame coincides with one of the path reference frames.

6. Process for the reconstruction of three-dimensional images according to any one of the claims 1 to 5, characterized in that the paths are concentric.

7. Process for the reconstruction of three-dimensional images according to any one of the claims 1 to 6, characterized in that the planes are defined in the path and object reference frames by characteristic points belonging to these planes, the characteristic points associated with the path and object reference frames being different, and in that the pooling operations involve a conversion between the coordinates in the path reference frame (X1, Y1, Z1) of the characteristic point (M1) associated with said path reference frame (R1) and the coordinates (X3, Y3, Z3) in the object reference frame (R3) of the characteristic point (M3) associated with the object reference frame.

8. Process for the reconstruction of three-dimensional images according to claim 7, characterized in that, following conversion, the pooling operations involve forming a mean of the function sums associated with the different paths for certain characteristic points associated with the object reference frame.

9. Process for the reconstruction of three-dimensional images according to any one of the claims 1 to 7, characterized in that the radiation is an attenuation radiation emitted by a point source (4) at the focal point, rotating about the object in accordance with a circular source path.

10. Process for the reconstruction of three-dimensional images according to any one of the claims 1 to 7, characterized in that the radiation is an emission radiation from the object, the focal point being a point towards which the measuring directions of the sensors converge.

11. Process for the reconstruction of three-dimensional images according to any one of the claims 1 to 10, characterized in that the object reference frame has an axis corresponding to one direction of the interconnection describing the three-dimensional image of the object.

12. Apparatus comprising at least one bidimensional array of sensors, the arrays being displaced about an object to be examined in accordance with at least two circular paths and connected to measurement acquisition and processing means appropriate for performing a process for the reconstruction of three-dimensional images according to any one of the preceding claims.

FIG. 1

# FIG. 2

ART ANTERIEUR

# FIG. 3

FIG. 4

FIG. 6

FIG. 5